(19) Europäisches Patentamt
European Patent Office
Office européen des brevets

(11) **EP 3 895 695 A1**

(12) **EUROPEAN PATENT APPLICATION**

(43) Date of publication:
**20.10.2021 Bulletin 2021/42**

(21) Application number: **20382302.6**

(22) Date of filing: **15.04.2020**

(51) Int Cl.:
*A61K 9/10* (2006.01)     *A61K 9/107* (2006.01)
*A61K 36/48* (2006.01)     *A61K 36/484* (2006.01)
*A61K 36/53* (2006.01)     *A61K 47/44* (2017.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universidad Autónoma de Madrid
28049 Madrid (ES)**

(72) Inventors:
• **FORNARI REALI, Tiziana
28049 Madrid (ES)**
• **MARTÍN GARCÍA, Diana
28049 Madrid (ES)**

• **QUINTANA MARTINEZ, Somaris Elena
28049 Madrid (ES)**
• **REGLERO RADA, Guillermo
28049 Madrid (ES)**
• **RODRÍGUEZ GARCÍA-RISCO, Mónica
28049 Madrid (ES)**
• **VILLANUEVA-BERMEJO, David
28049 Madrid (ES)**

(74) Representative: **Hoffmann Eitle
Hoffmann Eitle S.L.U.
Paseo de la Castellana 140, 3a planta
Edificio LIMA
28046 Madrid (ES)**

Remarks:
Claims 16-19 are deemed to be abandoned due to non-payment of the claims fees (Rule 45(3) EPC).

(54) **SUPERCRITICAL CARBON DIOXIDE-BASED METHODOLOGY TO FORMULATE BIOACTIVE PREPARATIONS**

(57)     This invention pertains to the chemical field, in particular, to the field of pharmaceutical or galenic formulation, more particularly, it relates to methods to formulate homogenous dispersions and emulsions of bioactive substances in oily matrixes.

EP 3 895 695 A1

**Description**

**Technical field of the invention**

This invention pertains to the chemical field, in particular, to the field of pharmaceutical, nutraceutical, cosmetic or galenic formulation, more particularly, it relates to methods to formulate homogenous dispersions of bioactive substances in oily matrixes.

**Background of the invention**

Many bioactive components have been identified and isolated from vegetables, fruits, legumes, seeds, nuts, and whole grains, and have shown numerous beneficial effects on human health including antioxidant, anti-inflammatory, antibacterial, antitumor and immunomodulatory activities. The incorporation of these bioactive molecules into functional food ingredients and nutraceuticals, preserving their biological activity, attaining high bioavailability, and thus efficacy, is a challenging task due to the usual poor stability and/or low solubility of these biomolecules.

The main disadvantage of traditional formulation methods, such as those using encapsulating biopolymers (single emulsion, double emulsion, coacervation processes), electrospinning or spry drying, is the use of organic solvents where most of them are toxic. These solvents must be completely eliminated from the bioactive designed system before their intake, what demands heating steps that can lead to the degradation of the bioactive compounds.

Alternative methodologies for the formulation of bioactive molecules from vegetal matrix include the use of supercritical carbon dioxide ($SCCO_2$) technology. The application of supercritical fluids enables the control of the size and morphology of the bioactive particles. Moreover, $SCCO_2$ processes can be carried out at low temperatures in absence of oxygen. Several methods have been developed for the synthesis of micro and nano-particles based on spray processes using $SCCO_2$ either acting as a solute, a solvent or an antisolvent. In the literature, different type of food components, such as proteins, lipids, carbohydrates and other minor components were formulated using $SCCO_2$ technologies to target specific properties and functionality.

For example, the Particles from Gas-Saturated Solutions (PGSS) method is a well-known process in which the $SCCO_2$ acts as a solute. The process can make particles of materials that can absorb $SCCO_2$ at high concentrations. The supercritical fluid is dissolved in the molten material (e.g. a polymer or a liquid-suspended solution) and the high-pressure mixture is rapidly depressurized through a nozzle leading to particle formation by precipitation. PGSS is especially useful for the impregnation of active ingredients in polymer matrices.

In the case of Rapid Expansion of Supercritical Solutions (RESS) or Crystallization from Supercritical Solutions (CSS) applications, the particle formulation is carried out using $SCCO_2$ acting as solvent. These techniques are adequate when the biomolecules have high solubility in the supercritical fluid. The biomolecules are dissolved in $SCCO_2$ and the high-pressure solution is rapidly depressurized through a nozzle, to lead the precipitation of the biomolecules at low pressure.

Special attention has been carried out in last decades in supercritical particle formation technologies in which $SCCO_2$ acts as an antisolvent, with more than 10 diverse approaches developed. For example, in the Supercritical Anti-solvent (SAS) approach, the biomolecules have low solubility in $SCCO_2$ but high solubility in a liquid solvent which in turn is highly soluble in $SCCO_2$. A solution containing the biomolecules is sprayed through a nozzle into a chamber containing $SCCO_2$ that acts as an antisolvent. The rapid contact between the two phases generates supersaturation of the droplets, and results in fast nucleation and growth of solid particles. Another example in which $SCCO_2$ acts as an antisolvent is the Supercritical Fluid Extraction of Emulsions (SFEE). SFEE is an emulsion-based precipitation technique, which utilizes $SCCO_2$ as drying agent to extract the organic phase of an emulsion containing the bioactive compound. In this technique, a water-insoluble solute is previously dissolved in an organic solvent and the solvent is mixed with water to form a conventional emulsion. The organic solvent (dispersed phase of the emulsion) is subsequently extracted by $SCCO_2$ causing the supersaturation of the solute and the formation of particles, which are collected as a suspension in water. The combination of controlled emulsion droplet sizes and the fast precipitation kinetics caused by $SCCO_2$ leads to the formation of particles with controlled size and morphology. The addition of an encapsulating agent to the emulsion allows the formation of composites containing the solute. Moreover, different nozzles to inject the emulsion into the high-pressure chamber can be used to improve the contact between the solution and $SCCO_2$.

These supercritical particle formation technologies have been intensively applied in the area of pharmacy, while natural or food materials-based applications have been more limited although are growing currently due to their great potential. Lipids (β-sitosterol, soy lecithin, milk fat, cocoa butter), proteins (gelatin, lysozyme, zein) and carbohydrates (lactose, chitosan, β-glucan), are examples of food-related applications of $SCCO_2$ particle formation technologies. Indeed, the majority of recent studies are focused on minor component ingredients present in plant materials.

For example, CSS was used to encapsulate anthocyanins extracted for jabuticaba in order to increase and retain the pigments stability, protecting this compound from environmental conditions [9] and vanillin was micronized by RESS process [10] without altering its properties and holding its crystalline form. Furthermore, $SCCO_2$ has been utilized as antisolvent (SAS process) in a wide variety of process, such as the micronization of vitexin, an apigenin flavone glucoside found in the passion flower [11], the precipitation of high catechin/low caffeine powder from green tea [12], phenolic compounds from yarrow [13] or rosemary [14], among others.

There are numerous challenges circumvented with the different supercritical particle formation processes described and applied in the prior art. Specifically, one particular problem is that none of these prior art processes is directed to the supercritical dispersion of micronized particles in a lipid matrix, leading to a high performance formulation technique to combine the bioactivity of minor plant-based compounds and lipids, improving bioavailability and efficacy.

## Brief description of the figures

**Figure 1.** Scheme of BIOSAS process.

**Figure 2.** Schematic diagram of equipment used to accomplish BIOSAS process. ABPR: Automatic back pressure regulator, BPR: manual back pressure regulator, P: manometer, T: temperature probe, FC: flowmeter.

**Figure 3.** Backscattering spectra of the lipid dispersions (a), turbiscan stability index (b), microscopic imagen (c), and particle size distribution (d) of quercetin nano-particles dispersed in flaxseed oil after 72 h of BIOSAS production (experiment 1 in Table 1).

**Figure 4.** Microscopic imagen and particle size distribution of rosemary phytochemicals dispersed in (a) olive oil, (b) argan oil and (c) flaxseed oil (experiments 2, 8 and 9 in Table 1).

**Figure 5.** Backscattering spectra (a) and turbiscan stability index (b) of the lipid dispersions of rosemary extract obtained at 15 MPa and 313 K after 72 h of BIOSAS production (experiment 5 in Table 1) (a) and turbiscan stability index (b).

**Figure 6.** Melting curves (obtained by DSC) of olive oil and RES dispersions at 15 MPa and 313 K. Exp. 4: 100 g olive oil; Exp. 5: 50 g olive oil.

**Figure 7.** Microscopic imagen and particle size distribution of licorice root nanoparticles dispersed in flaxseed oil obtained at 20 MPa and 313.15 K after 48 h of BIOSAS production (experiment 12 in Table 1).

**Figure 8.** Backscattering spectra (a) and turbiscan stability index (b) of licorice root extract dispersed in flaxseed oil obtained at 20 MPa and 313.15 K after 48 h of BIOSAS production (experiment 12 in Table 1).

**Figure 9.** Optical microscope imagens of 20:80 W/O emulsions of *Quillaja saponaria* aqueous extract 48 h after their production: (a) 20 MPa and 1% lecithin, (b) 20 MPa and 1.25% lecithin, (c) 25 MPa and 1% lecithin and (d) 25 MPa and 1.25% lecithin.

**Figure 10.** Drop size distribution of 20:80 W/O emulsions of *Quillaja saponaria* aqueous extract 48 h after their production: (M1) 20 MPa and 1% lecithin, (M2) 20 MPa and 1.25% lecithin, (M3) 25 MPa and 1% lecithin, (M4) 25 MPa and 1.25% lecithin.

**Figure 11.** Effect of pressure and emulsifier (lecithin) in the mean droplet size of 20:80 W/O emulsions containing saponins produced by BIOSAS: (■) 20 MPa; (□) 25 MPa.

## Description of the invention

### Definitions

In the context of the present invention "solvent highly-soluble in SCCO2" is understood as an organic solvent which can be dissolved in SCCO2 in high concentration (at least 2-4 % mass) and preserving a homogeneous supercritical phase.

In the context of the present invention "homogeneously dispersed in the oily matrix with high stability" is understood as a regular and enduring distribution of small solid particles in oil.

In the context of the present invention "high-pressure vessel" is understood as a container that can withstand high pressure conditions (up to 500 bar).

In the context of the present invention "lipid phase" is understood as any type of lipid substance (liquid state).

In the context of the present invention "supercritical (SC) conditions" is understood as conditions of temperature and pressure above the critical temperature and pressure of CO2.

In the context of the present invention, "polar bioactive molecules" are understood as any single polar compound or multicomponent substance comprising polar compounds with proved biological activity.

In the context of the present invention, "water-solvent mixtures" are understood as mixtures of water with a solvent highly-soluble in $SCCO_2$.

## Description

In the present invention, we herein describe a new supercritical based methodology (from hereinafter referred to as BIOSAS) to incorporate bioactive compounds in a lipid matrix. The method is based in a SAS precipitation method to produce small drops of a solution (containing the bioactive molecules) in a high-pressure cell, in which the lipid matrix was placed. If the solvent of the solution is highly-soluble in SCCO2, the antisolvent effect causes the production of solid micro or nanoparticles which are energetically mixed with the lipid matrix. If the solvent of the solution is water or water-solvent mixtures, a water-oil emulsion is obtained. Therefore, the present invention is directed to the supercritical procedure BIOSAS to homogeneously incorporate or disperse bioactive substances in an oily matrix.

In the present invention, and as shown in the examples, it is herein demonstrated that BIOSAS can be successfully applied to disperse both pure bioactive substances (e.g. **quercetin**) as well as complex mixtures of bioactive substances (e.g. plant extracts). The solution provided implies pumping such bioactive substances into the system of the present invention, wherein such bioactive substances are contained or carried in a solvent highly-soluble in SCCO2 (e.g. ethanol), in which case, once introduced into the system, solid micro- and nano-particles bioactive substances shall form and will be homogeneously dispersed in an oily matrix with high stability.

In particular, as indicated in example 2.1, the dispersion of a pure bioactive compound such as quercetin in flaxseed oil can be carried out, according to the present invention, by first loading a high-pressure vessel with the oil, optionally including a surfactant, then, pumping CO2 into the pressure vessel until supercritical (SC) conditions are reached; pumping a dissolution comprising a solvent highly-soluble in SCCO2 such as ethanol and quercetin into the high-pressure vessel through a nozzle; together with the SCCO2 flow set; and collecting the oil dispersion comprising the quercetin in small particles in the lipid phase. The resulted concentration of quercetin in the flaxseed oil was 1.55 $\pm$ 0.05 % mass (HPLC analysis). Almost 100% quercetin dispersion yield (quercetin in oil / quercetin pumped into the mixing vessel) was obtained, since no quercetin was possible to identify in the ethanol recovered in the separator. The solubility of quercetin in a medium chain triacylglycerol mixture at 393.15 K is 1.77 $\pm$ 0.29 mg/g. This value is considerably lower than the amount dispersed by BIOSAS process in high molecular weight triacylglycerols at noticeably lower temperature (> 15 mg/g). Several commercial products are available in the market with encapsulated quercetin that recommend doses intake of 500-1000 mg/day, what would be equivalent to an intake of 30-70 ml/day of the quercetin-enriched flaxseed oil obtained by the BIOSAS process.

In addition, as indicated in example 2.2, the dispersion of a mixture of bioactive compounds such as rosemary phytochemicals in an oil can be carried out, according to the present invention, by first loading a high-pressure vessel with the oil, optionally including a surfactant, then, pumping CO2 into the pressure vessel until supercritical (SC) conditions are reached; pumping a dissolution comprising a solvent highly-soluble in SCCO2 such as ethanol and rosemary phytochemicals into the high-pressure vessel through a nozzle; together with the SCCO2 flow set; and collecting the oil dispersion comprising the rosemary phytochemicals in small particles in the lipid phase. This experiment was carried out with three different oils (Table 1) placed into the mixing vessel: olive oil (50 or 100 g), argan oil (50 g) and flaxseed oil (50 g) at different pressures (12, 15 and 20 MPa) and 313.15 K. The ethanol solution was pumped for 60 min in all experiments. In this case, while some phytochemicals of rosemary ethanolic solution RES were precipitated and dispersed in the oil, some other phytochemicals (those soluble in the supercritical CO2 + ethanol phase) were recovered with the ethanol cosolvent in the separator. In this way, two different fractions were obtained from RES, an oily product with some rosemary bioactives dispersed (RES-LP: lipid product) and a viscous fraction (RES-OP: oleoresin product) which was recovered from the separator after evaporation of ethanol. Table 2 shows the amount (mg) of rosemary phytochemicals recovered in RES-OP after ethanol evaporation. According to these values, it can be estimated that rosemary phytochemicals in the form of micro- and nano-particles were dispersed in olive oil in the range 0.15 % to 1.84 % mass, while in the case of argan and flaxseed oil these values were, respectively, 2.73% and 2.82 %. Thus, the dispersion of rosemary bioactives in argan oil or flaxseed oil was considerably larger than the in the case of olive oil. Under the conditions studied, the dispersion yields (phytochemicals in oil / phytochemicals pumped) attained in RES-LP were in the range of 4.72 to 56.50 % in the case of olive oil, while yields c.a. 85 % were obtained for argan and flaxseed oil.

However, interestingly, for comparative purposes with the BIOSAS process, some experiments were performed to dissolve the rosemary extract in olive oil at 313.15 K for 4 hours with continuous stirring. In this case, less than 0.5 % mass of the extract was possible to dissolve in the olive oil, after what precipitation of the extract occurred. Therefore, although no optimization was intended to carry out, BIOSAS permitted almost a 4-fold increase of a stable incorporation of rosemary phytochemicals in olive oil, in comparison with traditional dissolution process.

Furthermore, as indicated in example 2.3, the dispersion of a mixture of bioactive compounds such as licorice bioactive extracts in edible oils can be carried out, according to the present invention, by first loading a high-pressure vessel with the oil, optionally including a surfactant, then, pumping $CO_2$ into the pressure vessel until supercritical (SC) conditions are reached; pumping a dissolution comprising a solvent highly-soluble in $SCCO_2$ such as ethanol and licorice bioactive extracts into the high-pressure vessel through a nozzle; together with the $SCCO_2$ flow set; and collecting the oil dispersion comprising the licorice bioactive extracts in small particles in the lipid phase. Experiments were carried out with 70 g of three different oils (Table 1) placed into the mixing vessel: argan oil, flaxseed oil and olive oil. In all cases, successful dispersion of licorice phytochemicals in the oils was achieved, and high stability after 48 h of production was visually observed. It was estimated that c.a. 0.16, 0.26 and 0.18% of licorice bioactive microparticles were dispersed in argan, flaxseed and olive oil, respectively. BIOSAS dispersion yields (licorice phytochemicals in oil / licorice phytochemicals pumped into the mixing vessel) were in the range 7-20 %.

Lastly, as indicated in example 2.4, in the case of high polar bioactives, such as saponins, **water was the solvent used to produce Liquid A.** Since water has low solubility in $SCCO_2$, the micro- and/or nano-droplets of the solvent (water) containing the bioactives were dispersed in the lipid matrix (Liquid B) and thus, a W/O emulsion was obtained, being the water phase rich in the bioactive molecules. To favour the emulsion formation a food emulsifier (lecithin) was previously added to the lipid matrix.

In summary, in the present invention we have validated the BIOSAS supercritical technique. This SAS based process, BIOSAS, allows the control of particle sizes and allows the formulation of bioactive lipid-based products, such as nutraceuticals, functional food ingredients or food supplements, pharmaceutical, cosmetic or galenic formulations.

Therefore, a first aspect of the invention refers to a method to incorporate bioactive compounds in a lipid phase using supercritical carbon dioxide technology, wherein the method comprises:

a) Loading a high-pressure vessel with a lipid phase, optionally including a surfactant;
b) Pumping $CO_2$ into the pressure vessel until supercritical (SC) conditions are reached;
c) A dissolution comprising a solvent and a bioactive solute or a mixture of bioactive solutes, is pumped into the high-pressure vessel through a nozzle; together with the $SCCO_2$ flow set in step b); and
d) Collecting the lipid dispersion or emulsion formed in the lipid phase of step a).

It is noted that this process or methodology can be optionally performed in Continuous production as a flow production method used to manufacture, or produce the lipid dispersion or emulsion without interruption.

In a preferred embodiment of the first aspect of the invention, the lipid phase of step a) is selected from the list consisting of acylglycerides, fatty acids, fatty acid esters, squalene, alkylglycerols, phospholipids, sphingolipids, waxes, sterols, terpenes, terpenoids and mixtures thereof.

In another preferred embodiment of the first aspect of the invention, the solvent of step c) is selected from the list consisting of C1-C4 alcohols, preferable methanol and ethanol, isopropanol, methyl acetate, ethyl acetate, ethyl lactate, DMSO, acetone, and mixtures thereof.

In still another preferred embodiment, the solvent of step c) is selected from the list consisting of C1-C4 alcohols, preferable methanol and ethanol, isopropanol, methyl acetate, ethyl acetate, ethyl lactate, DMSO, acetone, and mixtures thereof and the lipid phase of step a) is selected from the list consisting of acylglycerides, fatty acids, fatty acid esters, squalene, alkylglycerols, phospholipids, sphingolipids, waxes, sterols, and mixtures thereof. Preferably, the dissolution of step c) comprises a solvent, which is very soluble in the $SCCO_2$, and a bioactive solute or a mixture of bioactive solutes, more preferably selected from the list consisting of an antioxidant, antimicrobial, antitumor, antiviral, anti-inflammatory, hypocholesterolemic, and hypotensive substances.

In another preferred embodiment of the first aspect of the invention, the liquid of step c) is a dissolution comprising water or water-solvent mixtures, and a bioactive solute or a mixture of bioactive solutes, more preferably selected from the above list.

In another preferred embodiment of the first aspect of the invention, the liquid of step c) is a dissolution comprising C1-C4 alcohols, preferable ethanol, and a bioactive solute or a mixture of bioactive solutes, more preferably selected from the list consisting of quercetin, rosemary bioactive compounds (rosmarinic acid, carnosic acid, carnosol), licorice bioactive compounds (liquiritin, liquiritigenin, glycyrrhizic acid, isoliquiritigenin, glabridin) and mixtures thereof.

In another preferred embodiment of the first aspect of the invention, the liquid of step c) is a dissolution comprising water or water-ethanol mixtures, and a bioactive solute or a mixture of bioactive solutes, more preferably selected from the list consisting of any type of saponins, rosmarinic acid and liquiritin and mixtures thereof.

In another preferred embodiment of the first aspect of the invention, the liquid of step a) is selected from the list consisting of olive oil, flaxseed oil, sunflower oil, fish oils and any type of edible oils, essential oils, argan oil and other type of cosmetic oils, triglycerides and mixtures thereof; and the solvent of step c) is selected from the list consisting of any solvent very soluble in SCCO2 such C1-C4 alcohols, preferable methanol and ethanol, isopropanol, methyl acetate, ethyl acetate, ethyl lactate, DMSO, acetone, and mixtures thereof.

In another preferred embodiment of the first aspect of the invention, the liquid of step a) is selected from the list consisting of olive oil, flaxseed oil, sunflower oil, fish oils and any type of edible oils, argan oil and other type of cosmetic oils, triglycerides and mixtures thereof; and the solvent of step c) is selected from the list consisting of water or water-ethanol mixtures.

In another preferred embodiment of the first aspect of the invention, the dissolution phase of step c) comprises the bioactive molecule or mixture of bioactive substances and the solvent in a weight ratio of 1:15 to 1:300, preferably of 1:30 to 1:160, and more preferably 1:50 to 1:110.

In another preferred embodiment of the first aspect of the invention, the dissolution phase of step c) and the SCCO2 are admixed in a weight ratio within the range of 1:4 to 1:120, preferably of 1:7 to 1:60, and more preferably 1:20 to 1:40.

In another preferred embodiment of the first aspect of the invention, the collected liquid phase of step c) has solid bioactive particles encapsulated of size of 0.1-5.0 $\mu$m, more preferably 0.4-1.3 $\mu$m or droplets encapsulated with a volume weighted average size of 5 to 200 $\mu$m, more preferably 45 to 110 $\mu$m.

A second aspect of the invention refers to a solid-liquid dispersion or a water-lipid emulsion in a lipophilic liquid obtainable by the method of the first aspect of the invention or of any of its preferred embodiments.

A third aspect of the invention refers to a lipid substance from the list consisting of olive oil, flaxseed oil, sunflower oil, fish oils and any type of edible oils, argan oil and other type of cosmetic oils, triglycerides and mixtures thereof, with solid bioactive particles homogeneously dispersed in the lipid phase, more preferably selected from the list consisting of quercetin, rosemary bioactive compounds (rosmarinic acid, carnosic acid, carnosol), licorice bioactive compounds (liquiritin, liquiritigenin, glycyrrhizic acid, isoliquiritigenin, glabridin) and mixtures thereof, characterized by a normal particle size distribution with mean size of 0.4-1.3 $\mu$m, and a mass concentration of bioactives of 0.2-3.0% (2-30 mg/g).

A fourth aspect of the invention refers to an oil-water emulsion in a weight ratio within the range of 10:90 to 30:70, more preferably 20:80, in which the water phase contain the bioactive solutes, more preferably selected from the list consisting of any type of saponins, rosmarinic acid and liquiritin and mixtures thereof, characterized by a drop size rather homogeneous and in the range 45-110 $\mu$m, and a mass concentration of bioactives of 0.2-3.0% (2-30 mg/g).

A fifth aspect of the invention refers to the solid-liquid dispersion or the water-lipid emulsion of the second aspect of the invention, the lipid substance with solid bioactive particles of the third aspect of the invention, or the oil-water emulsion of the fourth aspect of the invention, for use in therapy.

A sixth aspect of the invention refers to the non-therapeutic use of the solid-liquid dispersion or the water-lipid emulsion of the second aspect of the invention, the lipid substance with solid bioactive particles of the third aspect of the invention, or the oil-water emulsion of the fourth aspect of the invention, for applications in the field of cosmetics.

A pharmaceutical composition comprising the solid-liquid dispersion or the water-lipid emulsion of the second aspect of the invention, the lipid substance with solid bioactive particles of the third aspect of the invention, or the oil-water emulsion of the fourth aspect of the invention.

A cosmetic composition comprising the solid-liquid dispersion or the water-lipid emulsion of the second aspect of the invention, the lipid substance with solid bioactive particles of the third aspect of the invention, or the oil-water emulsion of the fourth aspect of the invention.

A nutraceutical composition or food drug composition comprising the solid-liquid dispersion or the water-lipid emulsion of the second aspect of the invention, the lipid substance with solid bioactive particles of the third aspect of the invention, or the oil-water emulsion of the fourth aspect of the invention.

The following examples are merely illustrative of the present invention but do not limit the same.

**Examples**

**Example 1. Materials and methods**

**1.1 Chemicals**

$CO_2$ (N38) was supplied from Carburos Metálicos (Madrid, Spain). Ethanol (99.5 % purity) was purchased from Panreac (Barcelona, Spain).

Carnosic acid (> 97% purity) was from Cymit (Cymit Quimica S.L., Barcelona, Spain). From Sigma-Aldrich (St. Louis, MO, USA), 2-(3,4-Dihydroxyphenyl)-3,5,7-trihydroxy-4H-1-benzopyran-4-one,3,3',4',5,6-Pentahydroxyflavone - Quercetin (> 95 % purity), quillaja bark (20% saponins), L-$\alpha$-lecithin, egg yolk, highly purified soy bean lecithin and

rosmarinic acid ($\geq$ 98% purity) were purchased. Orthophosphoric acid (85% purity) was purchased from Scharlab S.L. (Sentmenat, Spain) and acetonitrile (99.8% purity) from Macron (Poland). Extra virgin oil, virgin flaxseed and argan oils were purchased in local markets.

## 1.2 Preparation of bioactive solutions

2 grams of quercetin were dissolved in 200 ml of ethanol to obtain the quercetin ethanolic solution, and 2 grams of quillaja bark saponins were dissolved in 200 ml of milli-Q water to produce the saponin water solution.

In the case of plant extracts, rosemary (*Romarinus officinalis* L.) leaves and licorice (*Glycyrrhiza glabra* L.) roots were obtained from Murciana herbalist's (Murcia, Spain) and were ground using a Premill 250 hammer mill (Lleal S.A., Granollers, Spain). Then, ultrasound assisted extraction (Branson Digital Sonifier 550 model, Danbury, USA) with ethanol was accomplished. The extractions were carried out with a sample/ethanol ratio of 1:10 (w/v) for 15 min at constant temperature (25 °C). The liquid extracts were rotary evaporated under vacuum until the concentrations of solid solutes were 17 mg/mL and 10 mg/mL, respectively for the rosemary and licorice ethanolic solutions. These values were set according to previous studies concerning the SAS precipitation of ethanolic rosemary solutions [Quintana, S. E., Villanueva-Bermejo, D., Reglero, G., Garcia-Risco, M. R., & Fornari, T. (2019). Supercritical antisolvent particle precipitation and fractionation of rosemary (Rosmarinus officinalis L.) extracts. Journal of CO2 Utilization, 34(April), 479-489. https://doi.org/10.1016/j.jcou.2019.07.032] and licorice ethanolic solutions [Somaris E. Quintana, Diego Martin Hernández, David Villanueva-Bermejo, Mónica R. Garcia-Risco, Tiziana Fornari. Fractionation and precipitation of licorice (Glycyrrhiza glabra L.) phytochemicals by supercritical antisolvent (SAS) technique] to produce dry micronized powders.

## 1.3 Supercritical antisolvent precipitation of biomolecules in oily matrix (BIOSAS)

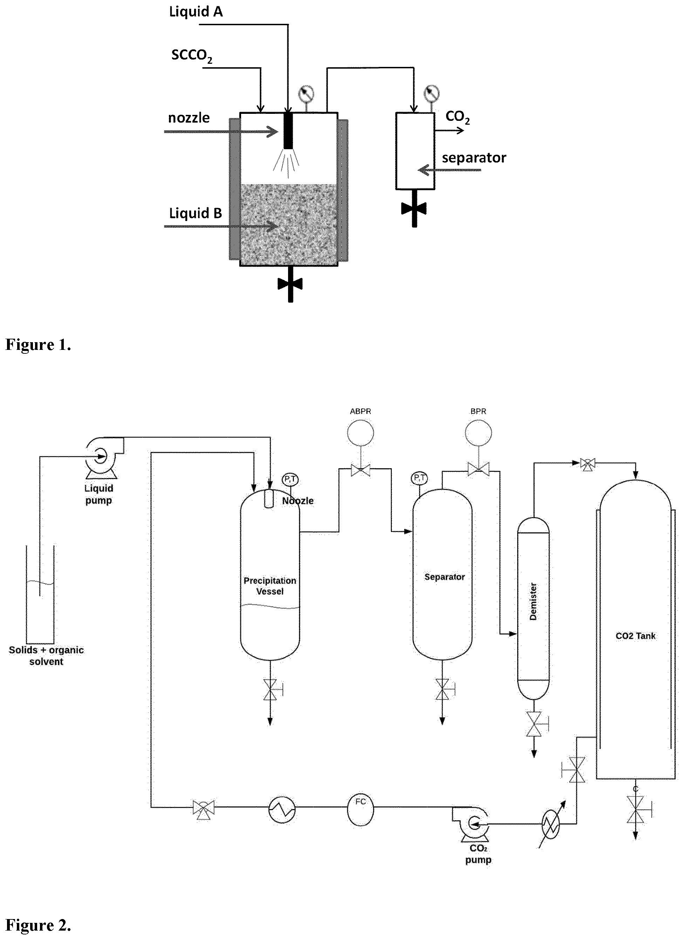

Figure 1 shows the scheme of the BIOSAS process. The solution containing the bioactive substances (Liquid A) is pumped through a nozzle into a mixing vessel, in which the oily matrix (Liquid B) was placed. Simultaneously, supercritical carbon dioxide (SCCO$_2$) is pumped into the vessel at high pressure and mild temperature.

When Liquid A is an ethanolic solution of bioactive substances, since the solvent is highly soluble in the supercritical fluid, the mass transfer of SCCO$_2$ into the ethanolic droplets produced by the nozzle, together with the evaporation of ethanol into the supercritical phase, lead to the supersaturation of the solutes dissolved in the droplets and produces their precipitation and simultaneous dispersion into Liquid B (oily phase). That is, solid micro- and/or nano-particles of bioactive molecules are produced, induced by the SCCO$_2$ antisolvent (SAS) effect and then, these particles are homogeneously dispersed in the oily matrix placed into the vessel. The supercritical outlet stream, which contains CO$_2$, ethanol and eventually some amount of bioactive molecules not dispersed, is decompressed and recuperated in a separator.

When Liquid A is a water solution, the low solubility of water in SCCO$_2$ hinder the antisolvent effect and thus the droplets (containing the bioactives) are mixed at high pressure conditions with the oily phase (Liquid B) producing water-oil (W/O) emulsions, providing if necessary the addition of a suitable amount of emulsifier in the oil. In this case, BIOSAS performance is similar to that of a high-speed homogenizer, and the addition of emulsifiers to Liquid B favours the stability of the formed emulsions.

BIOSAS process were performed using the supercritical technology equipment Thar SF2000 (Thar Technology, PA, USA) (Figure 2) with some modifications. The equipment comprises two high-pressure pumps for feeding, respectively, the supercritical SCCO$_2$ and Liquid A, and a stainless-steel mixing vessel (273 ml) and separator (500 ml) with independent control of temperature and pressure. The vessel is equipped with a 101.6 $\mu$m inner diameter nozzle for the injection of Liquid A. SCCO$_2$ and Liquid A streams are fed from the top in co-current manner (coaxial nozzle). A manual high-pressure valve is located at the bottom of the vessel to collect the oil with the dispersed solutes once BIOSAS process is completed. The supercritical outlet stream flows out of the vessel from the top. The equipment is coupled with a CO$_2$ recirculation system, including a demister unit, a filter, a condenser and a CO$_2$ storage tank.

SCCO$_2$ was pumped into the mixing vessel, where 50-100 g of oil was previously placed, until pressure (10-25 MPa) and temperature (308.15 or 313.15 K) conditions were attained. Then, the Liquid A was pumped for 10-60 min into the vessel at 1.6-3.2 ml/min, while maintaining a constant flow of SCCO$_2$ (50 g/min). In all the experiments, once the pumping of Liquid A through the nozzle was finished, SCCO$_2$ was pumped during additional 5-15 min. During the process, the separator was kept at 0.1 MPa and 313.15 K.

Particularly, in the case that Liquid A is an ethanolic solution, the depressurization of the supercritical stream in the separator produced the ethanol precipitation together with those substances which were not dispersed in Liquid B (i.e. solutes which are soluble in the SCCO$_2$-ethanol supercritical phase). This material was recovered from the separator and was rotary evaporated under vacuum until an oleoresin-type product was obtained. Finally, the vessel was depressurized, and the oily dispersion or W/O emulsion of bioactive substances was recovered by the high-pressure valve placed at the bottom of the mixing vessel. Products were kept at 298 K under darkness until analysis.

Table 1 shows all the experiments carried out in this invention to investigate BIOSAS practicability, varying the lipid matrix (Liquid B) and the pumped bioactive solution (Liquid A). Pressure and temperature, Liquid A and $SCCO_2$ flows, together with the amount of Liquid B placed in the mixing vessel and concentration of bioactives in Liquid A are given in the table.

**Table 1.** BIOSAS process conditions applied in all cases studied in this work: QUE: quercetin; RES: rosemary ethanolic solution; LES: licorice ethanolic solution; QWS: quillaja water solution.

| No. | Liquid A | Liquid A (mg/ml) | Liquid B | Liquid B (g in vessel) | P (MPa) | T (K) | Liquid A flow (ml/min) | Time (min) |
|---|---|---|---|---|---|---|---|---|
| 1 | QUE | 10 | flax oil | 57 | 20 | 313.15 | 2.0 | 45 |
| 2 | RES | 17 | olive oil | 100 | 12 | 313.15 | 1.6 | 60 |
| 3 | RES | 17 | olive oil | 50 | 12 | 313.15 | 1.6 | 60 |
| 4 | RES | 17 | olive oil | 100 | 15 | 313.15 | 1.6 | 60 |
| 5 | RES | 17 | olive oil | 50 | 15 | 313.15 | 1.6 | 60 |
| 6 | RES | 17 | olive oil | 100 | 20 | 313.15 | 1.6 | 60 |
| 7 | RES | 17 | olive oil | 50 | 20 | 313.15 | 1.6 | 60 |
| 8 | RES | 17 | argan oil | 50 | 20 | 313.15 | 1.6 | 60 |
| 9 | RES | 17 | flax oil | 50 | 20 | 313.15 | 1.6 | 60 |
| 10 | LES | 10 | argan oil | 70 | 15 | 308.15 | 3.2 | 45 |
| 11 | LES | 10 | argan oil | 70 | 20 | 313.15 | 3.2 | 30 |
| 12 | LES | 10 | flax oil | 70 | 20 | 313.15 | 3.2 | 30 |
| 13 | LES | 10 | olive oil | 70 | 20 | 313.15 | 3.2 | 57 |
| 14 | QWS | 10 | olive oil (1.00% lecithin) | 70 | 20 | 313.15 | 1.6 | 10 |
| 15 | QWS | 10 | olive oil (1.25% lecithin) | 70 | 20 | 313.15 | 1.6 | 10 |
| 16 | QWS | 10 | olive oil (1.00% lecithin) | 70 | 25 | 313.15 | 1.6 | 10 |
| 17 | QWS | 10 | olive oil (1.25% lecithin) | 70 | 25 | 313.15 | 1.6 | 10 |

### 1.4 Assessment of BIOSAS dispersion yield

In the case of ethanolic solutions, the BIOSAS dispersion yield was evaluated as the total mass of phytochemicals incorporated in the lipid matrix (Liquid B) related to the mass of phytochemicals introduced in the system by pumping the ethanolic solution (Liquid A). The total mass of phytochemicals dispersed in the lipid matrix was estimated as the difference between the mass pumped and the mass of solids recovered in the separator (oleoresin), which was obtained by evaporation of the ethanol solvent. Thus, it is assumed that no loss of phytochemicals was produced (e.g. material detained in the tubing or dragged out of the system with the decompressed $CO_2$). Furthermore, in the case of rosemary solution, two key bioactives (carnosic acid and rosmarinic acid) were identified and quantified in the lipid matrix and in the oleoresin, in order to check mass balance and verify the hypothesis assumed.

### 1.5 HPLC analysis

Carnosic acid and rosmarinic acid were identified and quantified in the case of BIOSAS rosemary samples following the procedures described by G. Vicente, M.R. Garcia-Risco, T. Fornari, G. Reglero, Isolation of carsonic acid from rosemary extracts using semi-preparative supercritical fluid chromatography, J. Chromatogr. A. 1286 (2013) 208-215. doi:10.1016/j.chroma.2013.02.044 using a HPLC Prominence-i LC-2030C 3D Plus (Shimadzu) equipped with a RP-C18 (250 × 4.6 mm; 3 μm) chromatography column. The mobile phase consisted of 0.1 % of phosphoric acid in water (solvent A) and acetonitrile (solvent B) applying the following gradient: 0-8 min, 77 % A, 8-25 min, 25 % A, 25-40 min 25 % A and the 40-45 min 77 % A. Initial conditions were gained in 5 min. The flow rate was constant at 0.7 ml/min.

Injection volume was 20 μl and the detection was accomplished using a diode array detection system, storing the signal at a wavelength of 230, 280 and 350 nm.

In the case of quercetin BIOSAS dispersion, HPLC analysis was carried out as previously described by Wei et al. [17]. A Prominence-i LC-2030C 3D Plus (Shimadzu) equipped with a quaternary solvent delivery system, an autosampler and DAD detector and RP-C18 (250 × 4.6 mm; 3 μm) was used. The column temperature was set at 25 °C. The mobile phase consists of acetonitrile (A) and 0.026% aqueous H3PO4 (v/v), applying the following gradient elution: at 0-20 min, 20%-25% A, 20-30 min, 25%-34% A, 30-50 min, 34%-50% A, 50-60 min, 50%-60% A and 60-80 min, 60% A. After 5 min, the initial conditions were achieved. The flow rate was 0.7 ml/min and was kept constant during analysis. Injection volume was 20 μl and detection was accomplished at 254, 280 and 370 nm.

### 1.6 Morphology and particle size analysis

A confocal laser scanning microscope (Zeiss LSM 710, Carl Zeiss Micro imaging GmbH, Germany) was used to visualize the phytochemicals particles microstructure dispersed in oil. Phytochemicals particles were identified by simple polarized light. Carl Zeiss Plan-Apochromat 40× oil-immersion objective lens was employed to view each dispersion samples. Digital image files in 512 × 512-pixel resolution were recorded with the Zen LSM software (Carl Zeiss Micro imaging GmbH, Germany).

The size distribution of phytochemicals dispersed in the oils was measured by Zetasizer analyzer (Malvern Zetasizer Nano ZS, from Malvern Instruments Ltd - UK). The instrument was attached with a He-Ne laser lamp (0.4 mW) at a wavelength of 633 nm. Measurements were carried out at 25°C in an insulated chamber using dynamic light scattering technique. In the case of emulsions, particle size and particle size distributions were measured by light scattering with a laser diffraction system Mastersizer 2000 (Malvern Instruments Ltd., Malvern, UK), equipped with a wet dispersion unit.

### 1.7 Stability of the bioactive-rich oily dispersions

The physical destabilization of the oily dispersions against time was evaluated by measuring the settling behavior using the multiple light technique scattering (MLS) (Turbiscan Lab Expert, Formulaction, France) in which a pulsed near-infrared light source with a wavelength of 850 nm is forced to pass through a suspension maintained at rest into a glass tube. In this work 8-10 ml of samples were measured; the data were recorded every 5 minutes for 2 h, 48 h after preparation.

Backscattering and Turbiscan stability index (TSI) were used to quantify the stability of samples:

$$TSI = \sum_l \frac{\sum_h |scan_l(\text{h}) - scan_{l-1}(\text{h})|}{H}$$

Where, h and H is the selected height and the total height of sample respectively. This parameter accounts for all processes that occur in the sample (particle coalescence and settling processes). A higher TSI value indicates a less stable emulsion

For emulsions the zeta-potential of emulsions were measured at 25 °C using a Zetasizer analyzer (Malvern Zetasizer Nano ZS, from Malvern Instruments Ltd - UK). Before measurement, the samples were diluted at 5 % in distilled water.

### 1.8 Thermal properties

Thermal properties of samples were characterized by using differential scanning calorimetry (DSC-Q200 TA Instruments, USA). The samples were heated from 25 °C to 200 °C using an aluminum crucible with a pierced lid with a 1.0 mm diameter orifice and approximately 4 mg of each sample under an air flow rate of 150 mL/min at a heating rate of 10 °C/min.

### 2. Results

Table 1 above shows the BIOSAS conditions applied in all experiments carried out for the present invention. It should be pointed out that no optimization of BIOSAS process was particularly intended in this invention for any specific study-case, but a good number of BIOSAS process applications were envisioned in order to demonstrate the potential of this novel supercritical procedure.

**2.1 BIOSAS dispersion of quercetin in oil**

The dispersion of a pure bioactive compound (quercetin) in oil was carried out first to assess the feasibility of a simple BIOSAS process to disperse a moderate polar compound within a pure lipid matrix. The conditions were 20 MPa, 313.15 K, with $SCCO_2$ and quercetin solution (QUE) flows of 50 g/min and 2 ml/min, respectively. These operating conditions attain a homogenous supercritical $CO_2$ plus ethanol phase at 313.15 K and guarantee the complete elimination of ethanol from the mixing vessel [S.N. Joung, C.W. Yoo, H.Y. Shin, S.Y. Kim, K.-P. Yoo, C.S. Lee, W.S. Huh, Measurements and correlation of high-pressure VLE of binary CO2-alcohol systems (methanol, ethanol, 2-methoxyethanol and 2-ethoxyethanol), Fluid Phase Equilib. 185 (2001) 219-230. doi:10.1016/S0378-3812(01)00472-1; C.J. Chang, C.-Y. Day, C.-M. Ko, K.-L. Chiu, Densities and P-x-y diagrams for carbon dioxide dissolution in methanol, ethanol, and acetone mixtures, Fluid Phase Equilib. 131 (1997) 243-258. doi:10.1016/S0378-3812(96)03208-6; Ž. Knez, M. Škerget, L. Ilic, C. Lütge, Vapor-liquid equilibrium of binary CO2-organic solvent systems (ethanol, tetrahydrofuran, ortho-xylene, meta-xylene, para-xylene), J. Supercrit. Fluids. 43 (2008) 383-389. doi:10.1016/J.SUPFLU.2007.07.020]. The concentration of quercetin in QUE was 10 mg/ml, and flaxseed oil (57 g) was the oily matrix placed into the mixing vessel. The QUE solution was pumped for 45 min.

The resulted concentration of quercetin in the flaxseed oil was 1.55 $\pm$ 0.05 % mass (HPLC analysis). High quercetin dispersion yield (quercetin in oil / quercetin pumped into the mixing vessel) was obtained, since no quercetin was possible to identify in the ethanol recovered in the separator. According to previous studies, the solubility of quercetin in a medium chain triacylglycerol mixture at 393.15 K is 1.77 $\pm$ 0.29 mg/g [21]. This value is considerably lower than the amount dispersed by BIOSAS process in high molecular weight triacylglycerols at noticeably lower temperature (> 15 mg/g). Several commercial products are available in the market with encapsulated quercetin that recommend doses intake of 500-1000 mg/day, what would be equivalent to an intake of 30-70 ml/day of the quercetin-enriched flaxseed oil obtained by the BIOSAS process.

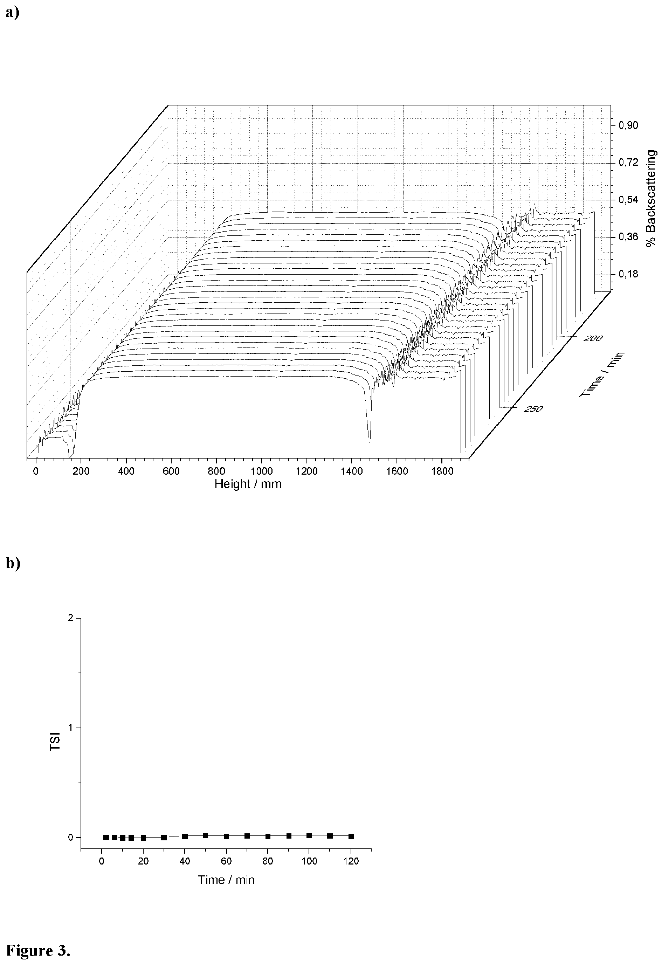

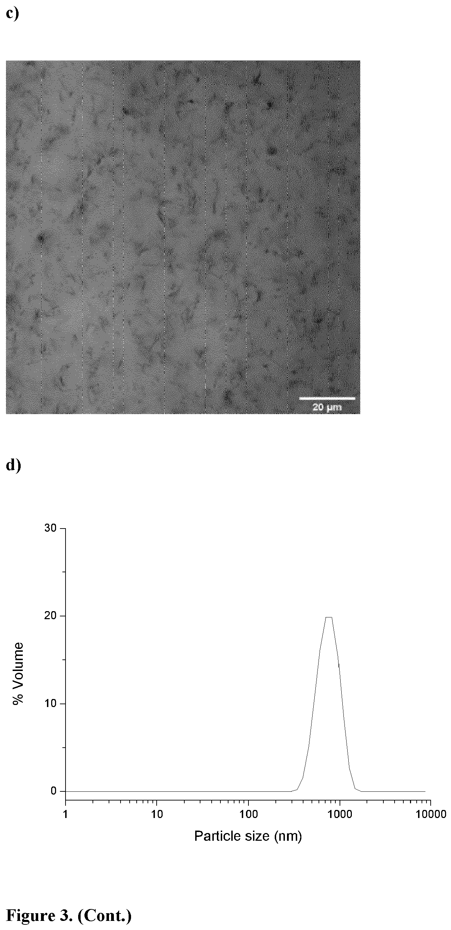

Figure 3 show the backscattering spectra of the lipid dispersions 72 h after BIOSAS production (Figure 3.a), the optical microscope image of the quercetin particles dispersed in the flaxseed oil (Figure 3.c) and the particle size distribution determined by light scattering (Figure 3.d). Multiple Light Scattering can detect, at early stage, the occurrence of different simultaneous destabilization mechanisms before they could be observed by the naked-eye. The backscattering profile of the quercetin - olive oil dispersion as a function of the storage time and the length of the container (a tube) is depicted in Figure 3.a. As can be observed in the figure, the profile did not show significant changes in intensity, indicating that the dispersion was stable until the time of analysis (72 h after production). Moreover, Figure 3.b shows the Turbiscan Stability Index (TSI) of the sample. The TSI parameter evaluates the stability of the sample by measuring its light intensity variations from the bottom to the top. The representation of TSI values vs. time of the flaxseed oil + quercetin dispersion is illustrated in Figure 3.b. During the studied time, the TSI values did not change significantly with time and were lower than 0.1.

Furthermore, quercetin nano-particles were homogeneously dispersed in flaxseed oil, with a normal particle size distribution and a mean particle size of 739.9 $\pm$ 188.4 nm (Figures 3.b and 3.c). Therefore, the BIOSAS process allowed the homogeneous and stable spreading of moderate polar compound within a non-polar matrix under the form of nano-particles. The dispersions obtained were stored in beaker tubes at ambient temperature and showed good stability after a week of production: no sediments and/or particle agglomerations were visually observed.

**2.2 BIOSAS dispersion of rosemary extract in oils**

The following example of application of the BIOSAS process was performed to assess the feasibility of this procedure to disperse a complex natural extract rich in different phytochemicals within a pure lipid matrix.

The flow rates of $SCCO_2$ and rosemary ethanolic solution (RES) were, respectively, 50 g/min and 1.6 ml/min. The concentration of rosemary extract in RES was 17 mg/ml. Experiments were carried out with three different oils placed into the mixing vessel (Table 1): olive oil (50 or 100 g), argan oil (50 g) and flaxseed oil (50 g) at different pressures (12, 15 and 20 MPa) and 313.15 K. The RES solution was pumped for 60 min in all experiments.

In this case, while some phytochemicals of RES were precipitated and dispersed in the oil, some other phytochemicals (those soluble in the supercritical $CO_2$ + ethanol phase) were recovered with the ethanol cosolvent in the separator. In this way, two different fractions were obtained from RES, an oily product with some rosemary bioactives dispersed (RES-LP: lipid product) and a viscous fraction (RES-OP: oleoresin product) which was recovered from the separator after evaporation of ethanol.

Table 2 shows the amount of rosemary phytochemicals recovered in RES-OP after ethanol evaporation. According to these values, it can be estimated that rosemary phytochemicals in the form of micro- and nano-particles were dispersed in olive oil in the range 0.15 % to 1.84 % mass, while in the case of argan and flaxseed oil these values were, respectively, 2.73% and 2.82 %. Thus, the dispersion of rosemary bioactives in argan oil or flaxseed oil was considerably larger than the in the case of olive oil. Under the conditions studied, the dispersion yields (phytochemicals in oil / phytochemicals pumped) attained in RES-LP were in the range of 4.72 to 56.50 % in the case of olive oil, while yields c.a. 85 % were obtained for argan and flaxseed oil.

**Table 2.** BIOSAS yield (phytochemicals in Liquid B / phytochemicals in Liquid A pumped) and concentration of phytochemicals (% mass) in the lipid matrix (Liquid B): QUE: quercetin; RES: rosemary ethanolic solution; LES: licorice ethanolic solution; QWS: quillaja water solution.

| No. | Liquid A | Liquid B | Phytochemicals recovered in the separator (mg) | BIOSAS yield (%) | Phytochemicals concentration in Liquid B (% mass) |
|---|---|---|---|---|---|
| 1 | QUE | flax oil | n. d. | - | 1.58 |
| 2 | RES | olive oil | 1310 | 19.73 | 0.32 |
| 3 | RES | olive oil | 1555 | 4.72 | 0.15 |
| 4 | RES | olive oil | 1240 | 24.02 | 0.39 |
| 5 | RES | olive oil | 1195 | 26.78 | 0.87 |
| 6 | RES | olive oil | 1160 | 28.92 | 0.47 |
| 7 | RES | olive oil | 710 | 56.50 | 1.84 |
| 8 | RES | argan oil | 265 | 83.76 | 2.73 |
| 9 | RES | flax oil | 220 | 86.52 | 2.82 |
| 10 | LES | argan oil | 1330 | 7.64 | 0.16 |
| 11 | LES | argan oil | 850 | 11.46 | 0.16 |
| 12 | LES | flax oil | 780 | 18.75 | 0.26 |
| 13 | LES | olive oil | 1700 | 6.80 | 0.18 |
| 14 | QWS | olive oil (1.00% lecithin) | - | - | 0.23 |
| 15 | QWS | olive oil (1.25% lecithin) | - | - | 0.23 |
| 16 | QWS | olive oil (1.00% lecithin) | - | - | 0.23 |
| 17 | QWS | olive oil (1.25% lecithin) | - | - | 0.23 |

n.d.: no detected

For comparative purposes with the BIOSAS process, additional experiments were performed to dissolve the rosemary extract in olive oil at 313.15 K for 4 hours with continuous stirring. In this case, less than 0.5 % mass of the extract was possible to dissolve in the olive oil, after what precipitation of the extract occurred. Therefore, although no optimization was intended to carry out, BIOSAS permitted almost a 4-fold increase of a stable incorporation of rosemary phytochemicals in olive oil, in comparison with traditional dissolution process.

Table 3 shows the concentration of rosmarinic acid and carnosic acid determined in the RES-LP samples obtained with olive oil, with values in the range of 0.05-0.23 mg rosmarinic acid and 0.03-0.21 mg carnosic acid per gram of olive oil. Moreover, it was determined c.a. 0.40 mg/g of rosmarinic acid and 4.90 mg/g of carnosic acid dispersed in argan oil, and 0.51 mg/g of rosmarinic acid and 6.39 mg/g of carnosic acid in flaxseed oil. Then, the concentrations of these key rosemary antioxidants in argan oil or flaxseed oil were considerably higher than the values obtained in the case of olive oil. According to EFSA [The EFSA Journal (2008) 721, 1-29.] the dietary exposure of carnosic acid estimated for adults would be between 500-1500 mg per day. Considering an intake of 50 g of any of the oils per day, the carnosic acid daily intake is below the toxic limits established by EFSA. Rosmarinic acid itself exhibits low toxicity ($LD_{50}$ in mice is 561 mg/kg for intravenous administration) and is not mutagenic but there are insufficient toxicity data from which a safe dose could be derived [The EFSA Journal; No. 3593, Vol. 12 (3), DOI: 10.2903/j.efsa.2014.3593].

Table 3. Rosmarinic acid (RA) and carnosic acid (CA) determined in RES-LP (lipid product) and recovered in RES-OP (oleoresin product) after BIOSAS dispersion in olive oil.

| Exp. | RES-LP (lipid product) | | | | RES-OP (oleoresin product) | | | | RA dispersion yield (%) | CA dispersion yield (%) |
|---|---|---|---|---|---|---|---|---|---|---|
| | RA (mg/g oil) | CA (mg/g oil) | RA (mg) | CA (mg) | RA (mg/g oleoresin) | CA (mg/g oleoresin) | RA (mg) | CA (mg) | | |
| 2 | 0.10 | 0.07 | 10.0 | 7.0 | 3.8 | 90.0 | 5.0 | 117.9 | 66.8 | 5.6 |
| 3 | 0.12 | 0.04 | 6.0 | 2.0 | 4.8 | 80.3 | 7.5 | 156.7 | 44.6 | 1.3 |
| 4 | 0.14 | 0.09 | 14.0 | 9.0 | 5-0 | 78.0 | 6.2 | 96.7 | 69.3 | 8.5 |
| 5 | 0.21 | 0.20 | 10.5 | 10.0 | 4.4 | 83.4 | 5.3 | 99.7 | 66.6 | 9.1 |
| 6 | 0.15 | 0.05 | 15.0 | 5.0 | 7.4 | 95.7 | 8.6 | 111.0 | 63.6 | 4.3 |
| 7 | 0.23 | 0.21 | 11.5 | 10.5 | 3.4 | 124.3 | 2.4 | 88.3 | 82.7 | 10.6 |

Table 3 also shows the concentration of rosmarinic acid and carnosic acid resulted from the HPLC analysis of the oleoresins (RES-OP) recovered in the separators. Taking into account the total mass of rosmarinic acid and carnosic acid quantified in the RES-LP and RES-OP samples, and in order to satisfy the mass balance of these substances, the concentration of these substances in the rosemary extract should be 0.8-1.4 % mass for rosmarinic acid and 6.1-7.6 % mass for carnosic acid. These values are quite in accordance with those determined by HPLC analysis in the rosemary extract (0.93 % mass of rosmarinic acid and 6.77 % mass of carnosic acid). Thus, the mass balance was satisfactory verified and the hypothesis that no losses of phytochemicals were produced was reasonable confirmed for these substances.

The dispersion yields of rosmarinic acid and carnosic acid (mass dispersed in the oil / mass pumped into the mixing vessel) were calculated and are given in Table 3. Rosmarinic acid yields (44-82 %) were significantly higher than carnosic acid yields (2-10 %). This result can be explained considering the higher solubility of carnosic acid in $SCCO_2$, particularly when ethanol is present as cosolvent [24], in comparison with rosmarinic acid which is a high-polar substance almost insoluble in $SCCO_2$. While carnosic acid is solubilized in the supercritical stream flowing out of the mixing vessel to the separator, rosmarinic acid is precipitated and dispersed in the oil placed in the mixing vessel. Thus, rosmarinic acid is rather recovered dispersed in the lipid matrix while carnosic acid is preferably recovered in the separator (oleoresin product). That is, BIOSAS process can attain in one step the fractionation and incorporation of selective biomolecules of plant extracts in lipid matrix.

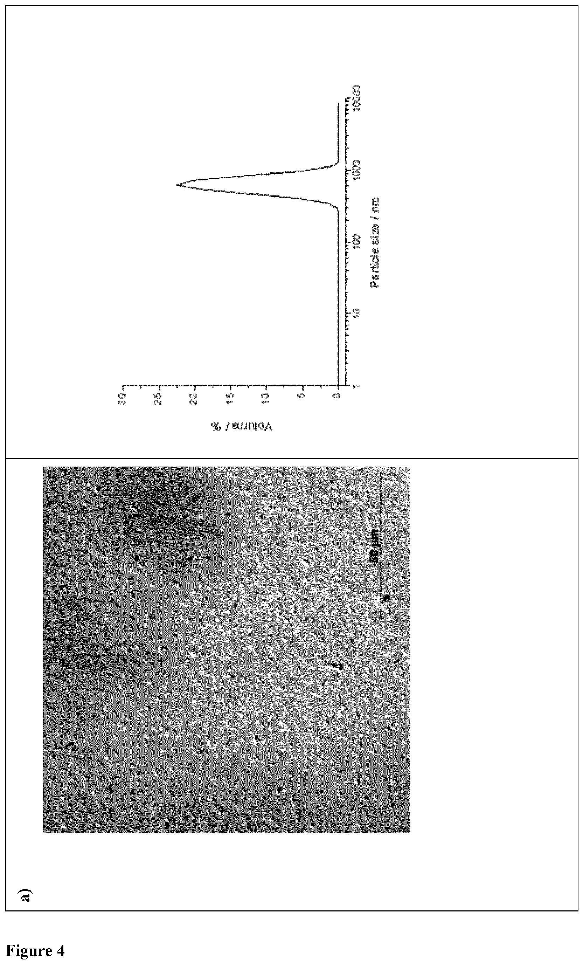

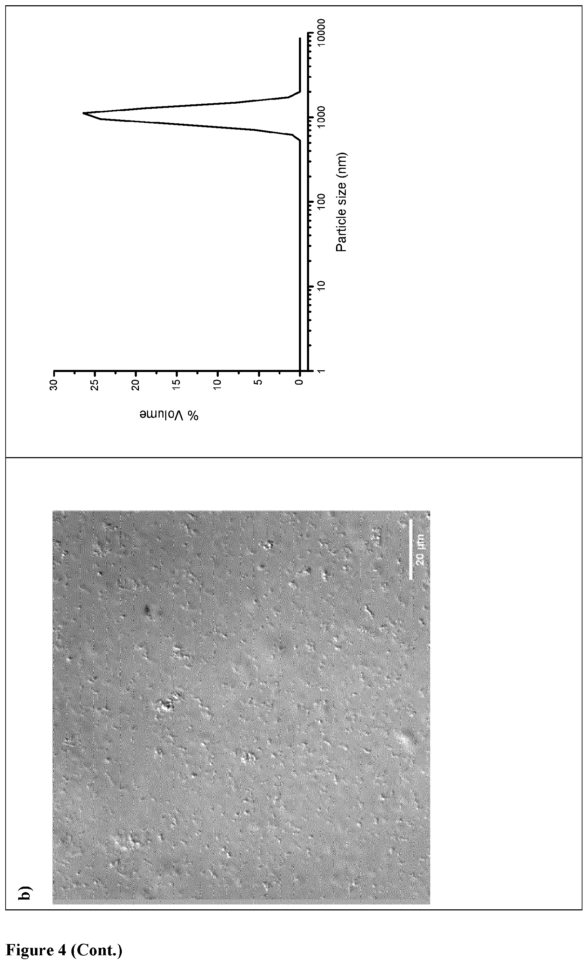

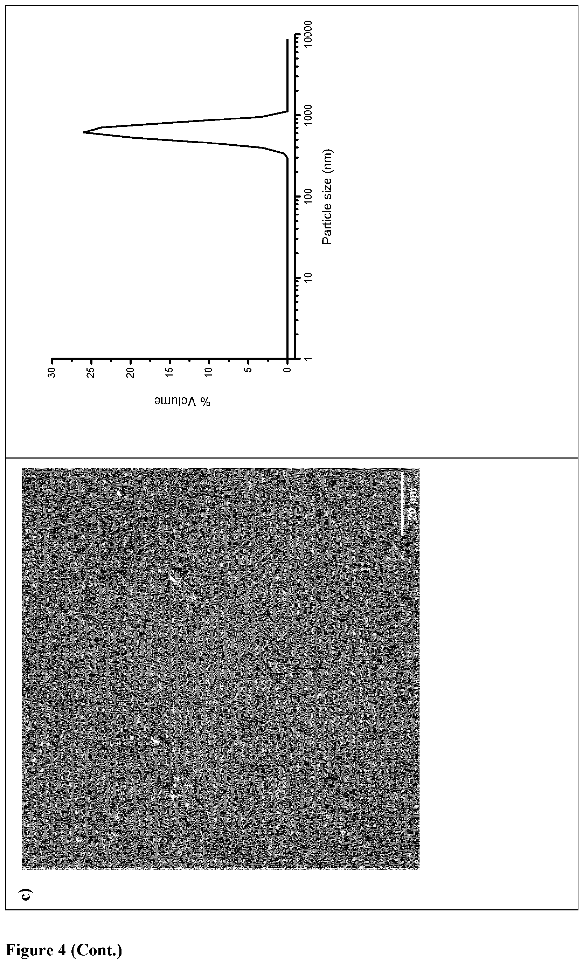

Figure 4 shows the microscopic imagen and particle size distribution of the rosemary dispersions obtained in the experiments 5 (olive oil), 8 (argan oil) and 9 (flaxseed oil) of Table 1. A homogeneous distribution of RES particles in the oils was achieved in all cases. The sizes of RES particles dispersed in the lipid matrix were below 1 $\mu$m, and in the case of olive oil the samples obtained at 15 MPa presented the lowest average size (397.85 $\pm$ 0.06 nm). Larger sizes were observed with argan oil and flaxseed oil, being the average size 1081 $\pm$ 224.6 nm and 621.91 $\pm$ 132.8 nm, respectively.

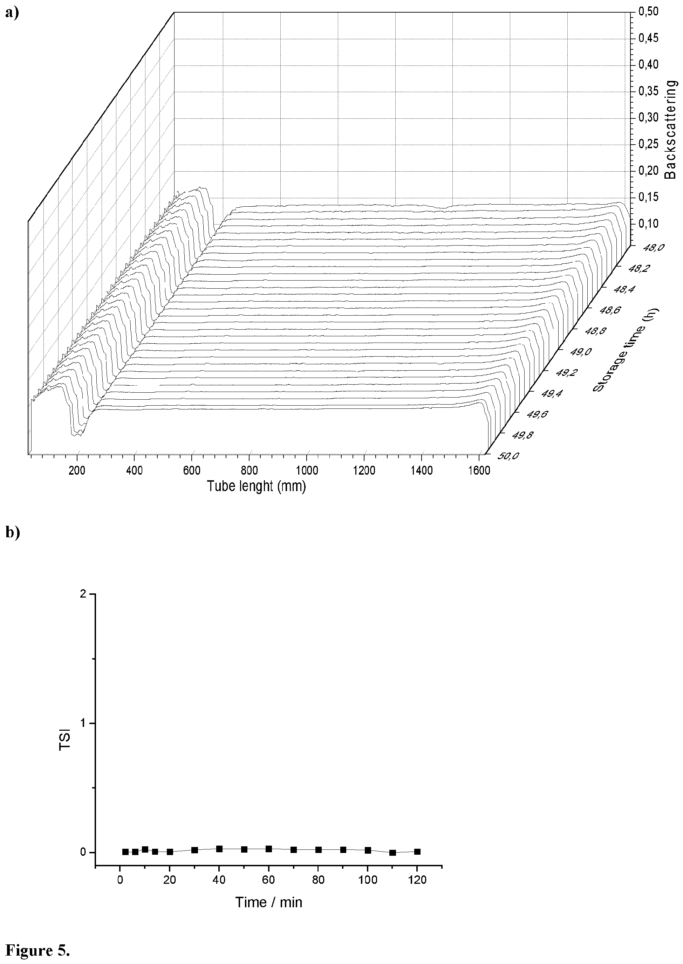

The dispersions obtained showed high stability after 48 h of production, since no particle migration by creaming or sedimentation and/or particle agglomeration was visually observed. Furthermore, the physical stability of the oil dispersions was characterized by Multiple Light Scattering. Figure 5 show the backscattering profile of the olive oil dispersion obtained in experiment 5 (50 g oil) as a function of the storage time and the length of the container (a tube). The backscatter profiles depicted in Figure 5 did not show significant changes in intensity indicating that the dispersions were stable until the time of analysis (48 h after production). Furthermore, the TSI values for this sample (Figure 5.b.) did not change significantly during time of analysis and values were lower than 0.1. Similar behavior was observed for the other rosemary dispersions.

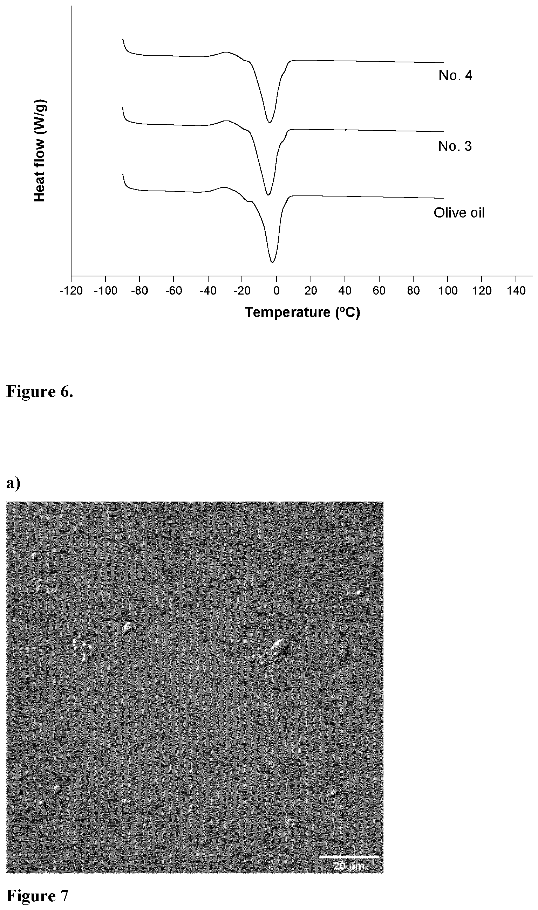

Thermal properties of the rosemary phytochemicals dispersed in olive oil were studied by differential scanning calorimetry (DSC). This technique offers a measure, as a function of temperature, of the heat flow (loss or gain) resulting from physical or chemical changes within a sample. It is usually used to characterize the crystallization and melting point behavior, giving information about polymorphism and/or crystal assembling. Figure 6 show the thermal behavior of the RES-olive oil dispersions obtained in experiments 4 and 5 of Table 1, in comparison with the pure olive oil. All the samples presented endothermic peaks and the thermal behaviors of both dispersions were very similar to that of olive oil, indicating similar thermal stability. The peak melting temperature of the dispersions 4 and 5 were 268.44 and 269.12 K, respectively, slightly lower than the melting point of olive oil (270.17 K) (Figure 6). This reduction in the peak melting temperature can be related to a less ordered structure, since lower amount of energy is required to breakdown the internal connections of the lipid matrix, indicating a more imperfect structure.

### 2.3 BIOSAS dispersion of licorice bioactive extract in edible oils

In order to extend the analysis of the viability of BIOSAS to incorporate plant phytochemicals to lipid matrix, the dispersion of a licorice root ethanolic extract was investigated. The flow rates of $SCCO_2$ and licorice ethanolic solution (LES) were, respectively, 50 g/min and 3.2 ml/min. The concentration of licorice extract in LES was 10 mg/ml. Experiments were carried out with 70 g of three different oils placed into the mixing vessel (Table 1): argan oil, flaxseed oil and olive oil. Process temperature, pressure and time are given in Table 1 for each of the four experimental assays accomplished. In all cases, successful dispersion of licorice e in the oils was achieved, and high stability after 48 h of production was visually observed. It was estimated that c.a. 0.16, 0.26 and 0.18% of licorice extract were dispersed in argan, flaxseed and olive oil, respectively. BIOSAS dispersion yields (licorice phytochemicals in oil / licorice phytochemicals pumped into the mixing vessel) were in the range 7-20 %.

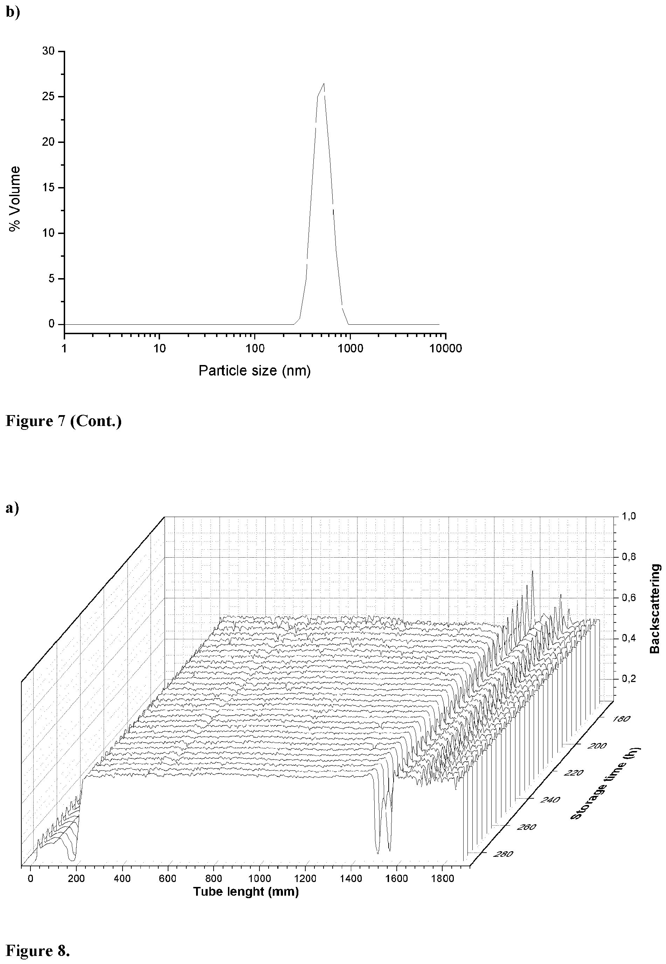

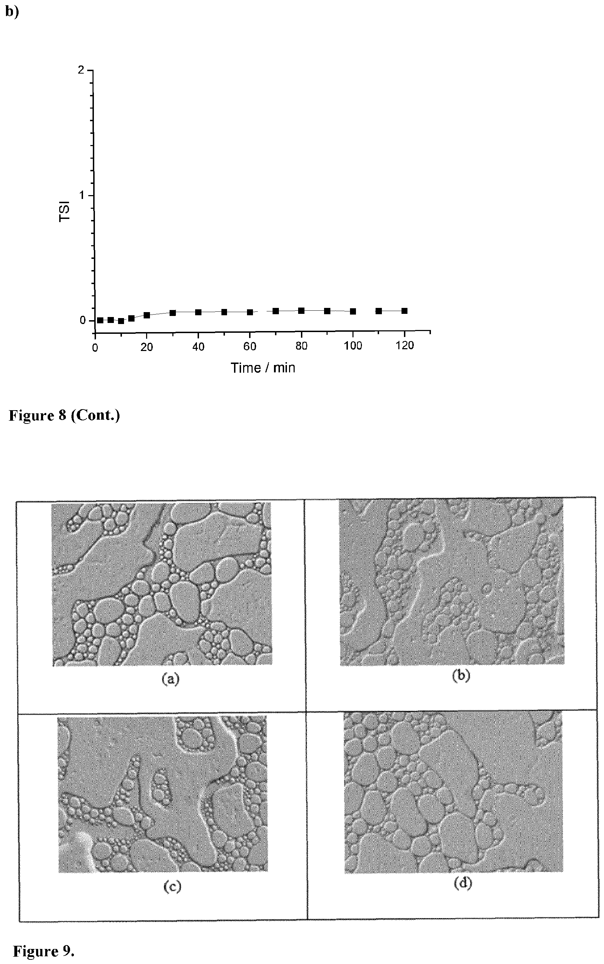

As an example, Figure 7 shows the microscopic imagen and particle size distribution of the licorice root dispersions obtained in the case of flaxseed oil (experiment 12 in Table 1). The sizes of LES particles dispersed in the lipid matrix were normal distributed, moderately narrow and monomodal in appearance, with average particle size of 451.9 $\pm$ 75.22 nm. Furthermore, the backscatter profiles depicted in Figure 8 did not show significant changes in intensity indicating that the dispersions were stable until the time of analysis (48 h after production). In the case of licorice phytochemicals dispersions, the TSI values showed somewhat higher variations with time of analysis, but also in these case values were always satisfactorily low (TSI < 0.13) indicating good stability (Figure 8.b.). Similar results were observed for the rest of LES dispersions obtained.

Therefore, the BIOSAS process allowed the homogeneous and stable spreading of complex natural extracts such as licorice or rosemary within oils under the form of nano-particles, but the dispersion yield being variable depending on the oil and the BIOSAS conditions.

## 2.4 BIOSAS emulsions

The potential of the BIOSAS method to disperse an aqueous solution of bioactives within a lipid was also tested in order to assess the feasibility of the BIOSAS process to produce stable emulsions as a form of formulation of bioactive compounds, but with the advantage of being performed under an inert atmosphere and at low temperatures. This would be of interest for formulations of unstable bioactive compounds within unstable lipid matrices that are susceptible to deterioration due to the own emulsifying conditions, such as the incorporation of air during the stirring or homogenization processes, or due to the high temperatures of some procedures, as high speed homogenization, high pressure homogenization or ultrasonication.

In this example, high polar bioactives such as saponins were tested, and water was the solvent used to produce Liquid A. Since water has low solubility in $SCCO_2$, the micro- and/or nano-droplets of the solvent (water) containing the bioactives are dispersed in the lipid matrix (Liquid B) and thus, a W/O emulsion is obtained, being the water phase rich in the bioactives. To favor the emulsion formation a food emulsifier (lecithin) was previously added to the lipid matrix.

The incorporation of an aqueous extract of *Quillaja saponaria* (QWS) in olive oil was carried out at 20-25 MPa, 313.15 K and 50 g/min of $SCCO_2$. The QWS (10 mg/g) was pumped at 1.6 ml/min for 10 min, while 70 g of olive oil was placed into the mixing vessel to achieve a c.a. 20:80 W/O emulsion. Two levels of lecithin (1.00 % and 1.25 %) were tested.

For all the experiments (14-17), all the QWS pumped was emulsified with the lipid matrix and thus, the concentration of quillaja extract in the olive oil after BIOSAS process was 0.23 % mass.

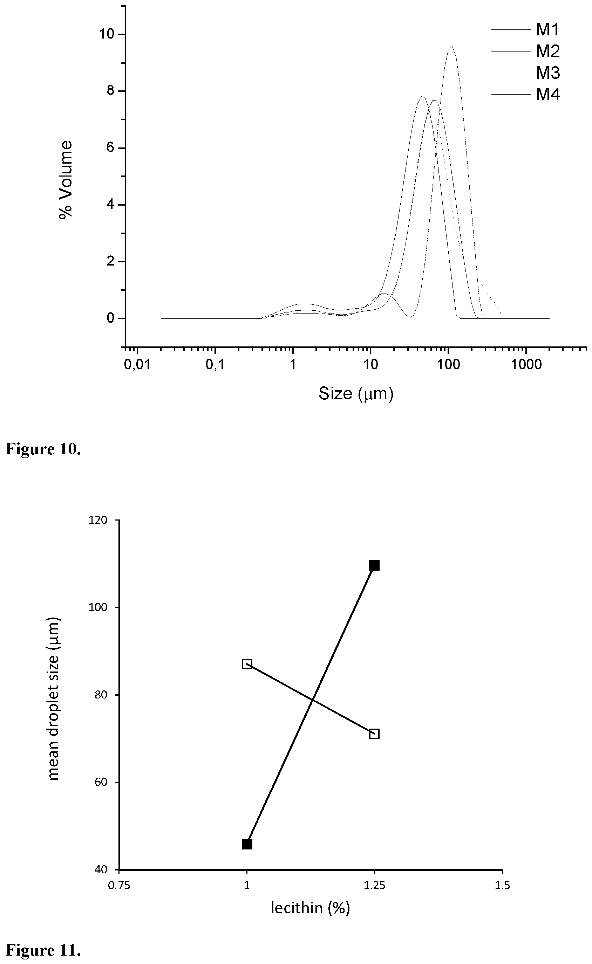

Figure 9 show the optical microscope imagens of the emulsions and Figure 10 the droplet size distribution. Droplet sizes are rather homogeneous and are in the range 10-200 $\mu$m (Table 4). The mean droplet sizes obtained, together with the zeta potential values after 48 h of production, are given in Table 5. The zeta potential values are close to -40 mV in all cases, indicating good stability of the emulsions obtained. Regarding the effect of pressure and lecithin content, Figure 11 shows a decrease in the mean droplet size with the decrease of lecithin content at 20 MPa, while the opposite effect was observed at 25 MPa. Despite more experiments are necessary to confirm this tendency, it seems that the effect of the emulsifier strongly depends on the mixing pressure used.

Therefore, the BIOSAS process allowed the formation of homogeneous and stable W/O emulsions to disperse high polar bioactive compounds as saponins within oils, but the droplet size of the bioactive aqueous solution depending on the combined effect of level of emulsifier and pressure of the process.

**Table 4.** Droplet sizes distribution ($\mu$m) of quillaja - olive oil W/O emulsions obtained by BIOSAS process.

| Exp. | d(0.1) | d(0.5) | d(0.9) |
|------|--------|--------|--------|
| 15 | 10.694 | 42.953 | 83.132 |
| 16 | 23.681 | 106.487 | 184.224 |
| 17 | 26.397 | 65.383 | 176.887 |
| 18 | 24.304 | 64.366 | 128.661 |

**Table 5.** Mean droplet diameter and zeta potential after 48 h of the BIOSAS production of quillaja - olive oil W/O emulsions.

| Exp. | Mean droplet size ($\mu$m) | Zeta potential (mV) |
|------|------|------|
| 14 | 45.83 | -43.50 $\pm$ 0.53 |
| 15 | 109.62 | -43.37 $\pm$ 2.54 |
| 16 | 87.04 | -43.23 $\pm$ 2.01 |
| 17 | 71.04 | -39.93 $\pm$ 1.80 |

**Claims**

1. A method to incorporate bioactive compounds in a lipid phase using supercritical carbon dioxide technology, wherein the method comprises:

   a) Loading a high-pressure vessel with a lipid phase, optionally including a surfactant;
   b) Pumping $CO_2$ into the pressure vessel until supercritical (SC) conditions are reached;
   c) A dissolution comprising a solvent and a bioactive solute or a mixture of bioactive solutes, is pumped into the high-pressure vessel through a nozzle; together with the $SCCO_2$ flow set in step b); and
   d) Collecting the lipid dispersion or emulsion formed in the lipid phase of step a).

2. The method of claim 1, wherein the liquid of step c) is a dissolution comprising a solvent, wherein the solvent is an organic solvent which can be dissolved in SCCO2 in a concentration of at least 2% w/w, and a bioactive solute or a mixture of bioactive solutes.

3. The method of claim 1, wherein the liquid of step c) is a dissolution comprising water or water-solvent mixtures, and a bioactive solute or a mixture of bioactive solutes.

4. The method of claim 2, wherein the liquid of step is selected from the list consisting of olive oil, flaxseed oil, sunflower oil, fish oils, essential oils, argan oil, triglycerides and mixtures thereof; and the solvent of step c) is selected from the list consisting of C1-C4 alcohols, preferable methanol and ethanol, isopropanol, methyl acetate, ethyl acetate, ethyl lactate, DMSO, acetone, and mixtures thereof.

5. The method of claim 3, wherein the liquid of step a) is selected from the list consisting of olive oil, flaxseed oil, sunflower oil, fish oils, essential oils, argan oil, triglycerides and mixtures thereof; and the solvent of step c) is selected from the list consisting of water or water-ethanol mixtures.

6. The method of any of claims 1, 2 or 4, wherein the bioactive solutes of step c) are selected from the list consisting of quercetin, rosemary bioactive compounds (rosmarinic acid, carnosic acid, carnosol), licorice bioactive compounds (liquiritin, liquiritigenin, glycyrrhizic acid, isoliquiritigenin, glabridin) and mixtures thereof.

7. The method of any of claims 1, 3 or 5, wherein the bioactive solutes of step c) are selected from the list consisting of saponins, rosmarinic acid, liquiritin and mixtures thereof.

8. The method according to any of claims 1, 2, 4 and 6, wherein if the solvent of the solution is soluble in SCCO2, the liquid of step c) comprises the bioactive molecule or mixture of bioactive substances and the solvent in a weight ratio of 1:15 to 1:300, preferably of 1:30 to 1:160, and more preferably 1:50 to 1:110.

9. The method according to any of claims 1, 2, 4, 6 and 8, wherein if the solvent of the solution is soluble in SCCO2, the liquid phase of step c) and the SCCO2 are admixed in a weight ratio within the range of 1:4 to 1:120, preferably of 1:7 to 1:60, and more preferably 1:20 to 1:40.

10. The method according to any of claims 1, 2, 4, 6, 8 and 9, wherein the collected liquid phase of step c) has solid bioactive particles encapsulated of size of 0.1-5.0 $\mu$m, more preferably 0.4-1.3 $\mu$m.

11. The method according to any of claims 1, 3, 5, 7, wherein the collected liquid phase of step c) has droplets encapsulated with a volume weighted average size of 5 to 200 $\mu$m, more preferably 45 to 110 $\mu$m.

12. A solid-liquid dispersion or a water-lipid emulsion in a lipophilic liquid obtainable by the method according to any of claims 1 to 11.

13. A lipid substance with solid bioactive particles homogeneously dispersed in a lipid phase, **characterized by** a lipid phase selected from the list consisting of olive oil, flaxseed oil, sunflower oil, fish oils, argan oil, triglycerides and mixtures thereof, with solid bioactive particles homogeneously dispersed in the lipid phase selected from the list consisting of quercetin, rosemary bioactive compounds (rosmarinic acid, carnosic acid, carnosol), licorice bioactive compounds (liquiritin, liquiritigenin, glycyrrhizic acid, isoliquiritigenin, glabridin) and mixtures thereof, **characterized by** a normal particle size distribution with mean size of 0.4-1.3 $\mu$m, and a mass concentration of bioactives of 0.2-3.0% (2-30 mg/g).

14. An oil-water emulsion in a weight ratio within the range of 10:90 to 30:70, more preferably 20:80, in which the water phase comprises bioactive solutes selected from the list consisting of any type of saponins, rosmarinic acid and liquiritin and mixtures thereof, **characterized by** a drop size in the range 45-110 $\mu$m, and a mass concentration ofbioactives of 0.2-3.0% (2-30 mg/g); and the lipid phase is selected from the list consisting of olive oil, flaxseed oil, sunflower oil, fish oils, argan oil, triglycerides and mixtures thereof,.

15. A solid-liquid dispersion or the water-lipid emulsion of claim 12, a lipid substance with solid bioactive particles of claim 13, or an oil-water emulsion of claim 14, for use in therapy.

16. A non-therapeutic use of the solid-liquid dispersion or the water-lipid emulsion of claim 12, the lipid substance with solid bioactive particles of claim 13 or the oil-water emulsion of claim 14, for applications in the field of cosmetics.

17. A pharmaceutical composition comprising the solid-liquid dispersion or the water-lipid emulsion of claim 12, the lipid substance with solid bioactive particles of claim 13, or the oil-water emulsion of claim 14.

18. A cosmetic composition comprising the solid-liquid dispersion or the water-lipid emulsion of claim 12, the lipid substance with solid bioactive particles of claim 13, or the oil-water emulsion of claim 14.

19. A nutraceutical composition or food drug composition comprising the solid-liquid dispersion or the water-lipid emulsion of claim 12, the lipid substance with solid bioactive particles of claim 13, or the oil-water emulsion of claim 14.

**Figure 1.**

**Figure 2.**

a)

b)

Figure 3.

c)

d)

**Figure 3. (Cont.)**

Figure 4

**Figure 4 (Cont.)**

Figure 4 (Cont.)

a)

b)

**Figure 5.**

**Figure 6.**

a)

**Figure 7**

b)

Figure 7 (Cont.)

a)

Figure 8.

b)

**Figure 8 (Cont.)**

**Figure 9.**

**Figure 10.**

**Figure 11.**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIU FUGUO ET AL: "Fabrication of Concentrated Fish Oil Emulsions Using Dual-Channel Microfluidization: Impact of Droplet Concentration on Physical Properties and Lipid Oxidation", JOURNAL OF AGRICULTURAL AND FOOD CHEMISTRY, vol. 64, no. 50, 21 December 2016 (2016-12-21), pages 9532-9541, XP055773963, US ISSN: 0021-8561, DOI: 10.1021/acs.jafc.6b04413 | 14,15 | INV.<br>A61K9/10<br>A61K9/107<br>A61K36/48<br>A61K36/484<br>A61K36/53<br>A61K47/44 |
| A | * abstract * | 1-13 | |
| A | SUN M ET AL: "Supercritical carbon dioxide extraction of carotenoids from carrot using canola oil as a continuous co-solvent", THE JOURNAL OF SUPERCRITICAL FLUIDS, ELSEVIER, AMSTERDAM, NL, vol. 37, no. 3, 1 May 2006 (2006-05-01), pages 397-408, XP024910194, ISSN: 0896-8446, DOI: 10.1016/J.SUPFLU.2006.01.008 [retrieved on 2006-05-01] * page 400, column 2, paragraph 2 * | 1-15 | |
| A | US 2016/331676 A1 (LAVILLE REMI [FR] ET AL) 17 November 2016 (2016-11-17) * paragraph [0122] * | 1-15 | |
| A | JP H09 157537 A (HASEGAWA T CO LTD) 17 June 1997 (1997-06-17) * paragraph [0009] * | 1-15 | |
| | -/-- | | |

TECHNICAL FIELDS
SEARCHED (IPC)

A61K

The present search report has been drawn up for all claims

| Place of search | Date of completion of the search | Examiner |
|---|---|---|
| Munich | 9 February 2021 | Sindel, Ulrike |

EPO FORM 1503 03.82 (P04C01)

page 1 of 2

Europäisches
Patentamt

European
Patent Office

Office européen
des brevets

**EUROPEAN SEARCH REPORT**

Application Number

EP 20 38 2302

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | QUINTANA SOMARIS E. ET AL: "Supercritical antisolvent particle precipitation and fractionation of rosemary (Rosmarinus officinalis L.) extracts", JOURNAL OF CO2 UTILIZATION, vol. 34, 1 December 2019 (2019-12-01), pages 479-489, XP055773163, ISSN: 2212-9820, DOI: 10.1016/j.jcou.2019.07.032 Retrieved from the Internet: URL:http://dx.doi.org/10.1016/j.jcou.2019.07.032> * the whole document * | 1-15 | |

-----

TECHNICAL FIELDS
SEARCHED      (IPC)

The present search report has been drawn up for all claims

| Place of search | Date of completion of the search | Examiner |
|---|---|---|
| Munich | 9 February 2021 | Sindel, Ulrike |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

page 2 of 2

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☒ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

## ANNEX TO THE EUROPEAN SEARCH REPORT
## ON EUROPEAN PATENT APPLICATION NO.

EP 20 38 2302

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2021

| Patent document cited in search report | Publication date | Patent family member(s) | Publication date |
|---|---|---|---|
| US 2016331676 A1 | 17-11-2016 | CN 105899186 A | 24-08-2016 |
| | | EP 3091960 A1 | 16-11-2016 |
| | | FR 3016289 A1 | 17-07-2015 |
| | | JP 6777546 B2 | 28-10-2020 |
| | | JP 2017502087 A | 19-01-2017 |
| | | PE 20160866 A1 | 24-09-2016 |
| | | US 2016331676 A1 | 17-11-2016 |
| | | WO 2015104484 A1 | 16-07-2015 |
| JP H09157537 A | 17-06-1997 | JP 3469696 B2 | 25-11-2003 |
| | | JP H09157537 A | 17-06-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **QUINTANA, S. E. ; VILLANUEVA-BERMEJO, D. ; REGLERO, G. ; GARCIA-RISCO, M. R. ; FORNARI, T.** Supercritical antisolvent particle precipitation and fractionation of rosemary (Rosmarinus officinalis L.) extracts. *Journal of CO2 Utilization,* April 2019, vol. 34, 479-489, https://doi.org/10.1016/j.jcou.2019.07.032
- **G. VICENTE ; M.R. GARCIA-RISCO ; T. FORNARI ; G. REGLERO.** Isolation of carsonic acid from rosemary extracts using semi-preparative supercritical fluid chromatography. *J. Chromatogr. A.,* 2013, vol. 1286, 208-215

- **S.N. JOUNG ; C.W. YOO ; H.Y. SHIN ; S.Y. KIM ; K.-P. YOO ; C.S. LEE ; W.S. HUH.** Measurements and correlation of high-pressure VLE of binary CO2-alcohol systems (methanol, ethanol, 2-methoxyethanol and 2-ethoxyethanol). *Fluid Phase Equilib.,* 2001, vol. 185, 219-230
- **C.J. CHANG ; C.-Y. DAY ; C.-M. KO ; K.-L. CHIU.** Densities and P-x-y diagrams for carbon dioxide dissolution in methanol, ethanol, and acetone mixtures. *Fluid Phase Equilib.,* 1997, vol. 131, 243-258
- **KNEZ, M. ŠKERGET ; L. ILIČ ; C. LÜTGE.** Vapor-liquid equilibrium of binary CO2-organic solvent systems (ethanol, tetrahydrofuran, ortho-xylene, meta-xylene, para-xylene). *J. Supercrit. Fluids.,* 2008, vol. 43, 383-389
- *The EFSA Journal,* 2008, vol. 721, 1-29
- *The EFSA Journal,* vol. 12 (3593), 3